# EUROPEAN PATENT APPLICATION

(11) **EP 1 363 125 A2**
(43) Date of publication of application: **19.11.2003**
(21) Application number: 03010388.1
(22) Date of filing: 08.05.2003
(51) Int. Cl.: G01N 33/543, C12Q 1/68, B01J 19/00, C12M 1/34

(54) **Transcription factor profiling on a solid surface**

(30) Priority: 08.05.2002 US 140956
(71) Applicant: Gentel Corporation, Madison, Wisconsin 53719 (US)
(72) Inventor: Nelson, Bryce, P., Madison, Wisconsin 53703 (US)
(74) Representative: Glawe, Delfs, Moll & Partner

(57) **Abstract**

The present invention relates to novel methods for the analysis of interactions of transcription factors with target nucleic acids. In particular, the present invention relates to compositions and methods for the detection of transcription factors binding to their target promoter regions. The present invention further provides methods of screening compounds for their ability to alter such binding interactions.

## Description

This application is a continuation in part of copending application serial number 10/140,956, filed 5/8/02, which is herein incorporated by reference in its entirety.

### FIELD OF THE INVENTION

The present invention relates to novel methods for the analysis of interactions of transcription factors with target nucleic acids. In particular, the present invention relates to compositions and methods for the detection of transcription factors binding to their target promoter regions. The present invention further provides methods of screening compounds for their ability to alter such binding interactions.

### BACKGROUND OF THE INVENTION

Transcription factors are proteins that bind to specific enhancer or promoter DNA sequences to regulate the transcription of certain genes. Under certain stimuli, transcription factors are created, or become active. To determine whether a given transcription factor is active or present in a sample, an electrophoretic mobility shift assay (EMSA) is often performed. In this procedure, a radioactively labeled DNA probe is mixed with a protein extract and the entire reaction is run on a nondenaturing polyacrylamide gel. Because the protein-bound probe will migrate more slowly than a free probe, the experiment is described as a "gel shift." Unfortunately, the gel shift is a cumbersome way to profile the activity of many transcription factors at once, because for every factor tested, a separate labeled probe needs to be generated. Moreover, results from such techniques are difficult to quantify.

Several commercially available replacements for the standard gel-shift assay exist. One method attaches the consensus DNA binding sequence to wells of a standard ELISA plate. Transcription factor binding is detected via an antibody-peroxidase conjugate, much like a standard ELISA detection. Examples include Active Motif's Paradigm Gel Shift and CloneTech's Mercury TransFactor kits. These kits can only detect transcription factors for which there is a corresponding antibody available. Panomics produces a transcription factor detection kit where fluorescently labeled double-stranded oligonucleotides are exposed to nuclear extracts. Protein-oligo complexes are purified, and the oligonucleotides that were bound by a transcription factor can be detected on an array. This detection method is indirect, can only observe binding to known DNA consensus sequences, and requires expensive, and variable, labeling of the DNA fragment.

What is needed are simple, cost effective methods for screening large numbers of transcription factor binding events.

### SUMMARY OF THE INVENTION

The present invention relates to novel methods for the analysis of interactions of transcription factors with target nucleic acids. In particular, the present invention relates to compositions and methods for the detection of transcription factors binding to their target promoter regions. The present invention further provides methods of screening compounds for their ability to alter such binding interactions.

For example, in some embodiments, the present invention provides a composition comprising an arrayed solid surface, the solid surface comprising an array of transcription factor binding targets. In some embodiments, the array of transcription factor binding targets comprises at least 20, preferably at least 50, even more preferably at least 100, and still more preferably, at least 1000 distinct target nucleic acid sequences. In some preferred embodiments, the solid surface is configured for label free detection. Accordingly, in some embodiments, the solid surface is an SPR surface (*e.g.,* an SPR prism). In some embodiments, the solid surface further comprises a plurality of microfluidics channels (*e.g.,* one dimensional or two dimensional arrays). In other embodiments, the solid surface further comprises a plurality of etched microchannels. In some embodiments, the transcription factor targets are double-stranded DNA molecules.

The present invention further provides a composition comprising an arrayed solid surface comprising a plurality of microfluidics channels, the solid surface comprising an array of transcription factor binding targets.

The present invention additionally provides a composition comprising an arrayed solid surface comprising a plurality of etched microchannels, the solid surface comprising an array of transcription factor binding targets.

The present invention also provides a composition comprising an arrayed solid surface comprising an array of transcription factor binding targets in contact with a biological sample containing at least one transcription factor.

The present invention further provides a composition comprising an arrayed solid surface comprising an array of transcription factor binding targets in contact with a biological sample containing at least one transcription factor, wherein the biological sample has been treated with a small molecule.

In still further embodiments, the present invention provides a composition comprising an arrayed solid surface comprising an array of transcription factors. In some embodiments, the array of transcription factors comprises at least 20, preferably at least 50, even more preferably at least 100, and still more preferably at least 1000 distinct transcription factors. In some preferred embodiments, the solid surface is configured for label free detection. Accordingly, in some embodiments, the solid surface is an SPR surface (*e.g.,* an SPR prism). In some embodiments, the solid surface further comprises a plurality of microfluidics channels (*e.g.,* one dimensional or two dimensional arrays). In other embodiments, the solid surface further comprises a plurality of etched microchannels.

The present invention further provides a composition comprising an arrayed solid surface comprising an array of transcription factors in contact with a sample comprising at least one transcription factor binding target.

The present invention also provides a composition comprising an arrayed solid surface comprising an array of transcription factors in contact with a treated biological sample, the treated biological sample used to prepare a cell lysate containing at least one test compound.

The present invention additionally provides a system comprising a composition comprising an arrayed solid surface, the solid surface comprising an array of transcription factor binding targets; at least one transcription factor polypeptide; and a detection apparatus in communication with the arrayed solid surface. In some embodiments, the array of transcription factor binding targets comprises at least 20, preferably at least 50, even more preferably at least 100, and still more preferably, at least 1000 distinct target nucleic acid sequences. In some embodiments, the at least one transcription factor polypeptide comprises at least 20, preferably at least 50, even more preferably at least 100, and still more preferably at least 1000 distinct transcription factors. In some embodiments, the system further comprises competitor DNA, wherein the competitor DNA has an identical nucleic acid sequence as the transcription factor binding targets. In other embodiments, the system further comprises an antibody that specifically recognizes the at least on transcription factor polypeptide. In some embodiments, the antibody is conjugated to a gold particle. In yet other embodiments, the system further comprises a second transcription factor target sequence, wherein a portion of the second transcription factor target sequence is complementary to the arrayed transcription factor target sequences. In still further embodiments, the system further comprises at least one test compound (*e.g.,* a drug). In some preferred embodiments, the solid surface is an SPR surface (*e.g.,* an SPR prism). In some embodiments, the solid surface further comprises a plurality of microfluidics channels (*e.g.,* one dimensional or two dimensional arrays). In other embodiments, the solid surface further comprises a plurality of etched microchannels. In some embodiments, the detection apparatus is configured for label-free detection. In other embodiments, the detection apparatus is configured for detection of a label.

The present invention further provides a system comprising a composition comprising an arrayed solid surface, the solid surface comprising an array of transcription factor polypeptides; at least one transcription factor binding target; and a detection apparatus in communication with the arrayed solid surface.

The present invention additionally provides a system comprising an arrayed solid surface, the solid surface comprising an array of transcription factor binding targets; and a biological sample containing at least one transcription factor, the biological sample in communication with the array solid surface.

The present invention also provides a system, comprising an arrayed solid surface, the solid surface comprising an array of transcription factor binding targets; and a biological sample containing at least one transcription factor, the biological sample in communication with the array solid surface, and wherein the biological sample has been treated with a small molecule.

The present invention further provides a system comprising a composition comprising an arrayed solid surface, the solid surface comprising an array of transcription factor polypeptides; at least one transcription factor binding target; and a cell lysate comprising at least one test compound, and the cell lysate in contact with the array solid surface.

In some embodiments, the present invention provides a method of detecting biomolecular interactions, comprising providing an arrayed solid surface, the solid surface comprising an array of transcription factor binding targets; at least one transcription factor polypeptide; and a detection apparatus in communication with the arrayed solid surface; and contacting the at least one transcription factor polypeptide with the array of transcription factor targets under conditions such that the apparatus detects interactions between the array of transcription factor targets and the at least one transcription factor polypeptide. In some embodiments, the array of transcription factor binding targets comprises at least 20, preferably at least 50, even more preferably at least 100, and still more preferably, at least 1000 distinct target nucleic acid sequences. In other embodiments, the array comprises one of the transcription factor binding targets. In some embodiments, the at lest one transcription factor polypeptide comprises at least 20, preferably at least 50, even more preferably at least 100, and still more preferably at least 1000 distinct transcription factors. In some embodiments, the method further comprises the step of contacting the at least one transcription factor polypeptide and the array of transcription factor targets with competitor DNA, wherein the competitor DNA has an identical nucleic acid sequence as the transcription factor binding targets. In some embodiments, the method further comprises the step of providing an antibody that specifically recognizes the at least on transcription factor polypeptide. In some embodiments, the antibody is conjugated to a gold particle. In some embodiments, the method further comprises the step of detecting the interaction between the transcription factor polypeptide and the transcription factor binding target by detecting the binding of the antibody to transcription factor polypeptide bound to the transcription factor binding target. In some embodiments, the method further comprises the step of providing a second transcription factor target sequence, wherein a portion of the second transcription factor target sequence is complementary to the arrayed transcription factor target sequences. In other embodiments, the method further comprises the step of, prior to the contacting, the step of contacting the second transcription factor target with the transcription factor polypeptide under conditions such that the second transcription factor target and the transcription factor polypeptide interact. In some preferred embodiments, the solid surface is an SPR surface (*e.g.,* an SPR prism). In some embodiments, the solid surface further comprises a plurality of microfluidics channels (*e.g.,* one dimensional or two dimensional arrays). In other embodiments, the solid surface further comprises a plurality of etched microchannels. In some embodiments, the detection apparatus is configured for label-free detection. In other embodiments, the detection apparatus is configured for detection of a label.

The present invention further provides a method of detecting biomolecular interactions, comprising providing an arrayed solid surface, the solid surface comprising an array of transcription factors; at least one transcription factor binding target; and a detection apparatus in communication with the arrayed solid surface; and contacting the at least one transcription factor binding target with the array of transcription factors under conditions such that the apparatus detects interactions between the array of transcription factors and the at least one transcription factor binding target.

The present invention also provides a method for measuring biomolecular interactions, comprising providing an arrayed solid surface, the solid surface comprising an array of transcription factor binding targets; a biological sample containing at least one transcription factor; and a detection apparatus; and contacting the biological sample with the arrayed solid surface targets under conditions such that the apparatus detects interactions between the array of transcription factor targets and the at least one transcription factor contained in the biological sample.

The present invention additionally provides a method for measuring the effect of small molecules on biomolecular interactions, the method comprising providing an arrayed surface, the surface comprising an array of transcription factor binding targets; a test compound; a biological sample comprising at least one transcription factor; a detection apparatus; and treating the biological sample with the small molecule; and contacting the treated biological sample with the array of transcription factor targets under conditions such that the apparatus detects interactions between the array of transcription factor targets and the at least one transcription factor contained in the biological sample. In some embodiments, prior to the step of contacting treated biological sample with the array of transcription factor, the biological sample is treated with a test compound. In some embodiments, the test compound is a drug. In some embodiments, the method further comprises the step of comparing the interactions between the array of transcription factor targets and the at least one transcription factor polypeptide in the presence of the test compound to the interactions in the absence of the test compound. In some embodiments, the test compound inhibits the interaction of the array of transcription factor targets and the at least one transcription factor polypeptide. In other embodiments, the test compound enhances the interaction of the array of transcription factor targets and the at least one transcription factor polypeptide.

The present invention further provides a method for measuring biomolecular interactions, comprising providing an arrayed solid surface, the solid surface comprising an array of transcription factors; a biological sample comprising at least one test compound; at least one transcription factor binding target; and a detection apparatus; and contacting the biological sample and the at least one binding target with the arrayed solid surface under conditions such that the detection apparatus detects interactions between the array of transcription factors and the at least one transcription factor binding target.

The present invention also provides a method for measuring the effect of small molecules on biomolecular interactions, comprising providing an arrayed solid surface, the surface comprising an array of transcription factor binding targets; at least one small molecule test compound; a biological sample containing at least one transcription factor; a detection apparatus; and treating the biological sample with the at least one small molecule test compound to generate a treated biological sample; contacting the treated biological sample with the array of transcription factor targets under conditions such that the apparatus detects interactions between the array of transcription factor targets and the at least one transcription factor contained in the biological sample.

### DESCRIPTION OF THE FIGURES

Figure 1 shows an overview of the creation of a photopatterned MUAM surface on an SPR-capable gold coated glass slide.
Figure 2 shows SPR data for the binding of AP2 DNA to AP2 protein. The top image shows real-time change in SPR signal measured on AP2 DNA target spots after addition of AP2 protein. The middle image shows the final difference image after AP2 binding to the arrayed AP2 oligonucleotides. The bottom image is a schematic representation of the array surface used in these experiments.
Figure 3 shows SPR data for AP2 protein binding in high salt concentration conditions.

### DEFINITIONS

As used herein, the term "solid surface" refers to any solid surface suitable for the attachment of biological molecules and the performance of molecular interaction assays. Surfaces may be made of any suitable material (*e.g.,* including, but not limited to, metal, glass, and plastic) and may be modified with coatings (*e.g.,* metals or polymers).

As used herein, the term "substrate" refers to any material with a surface that may be coated with a film.

As used herein, the phrase "coated with a film" in regard to a substrate refers to a situation where at least a portion of a substrate surface has a film attached to it (*e.g.* through covalent or non-covalent attachment).

As used herein, the term "microarray" refers to a solid surface comprising a plurality of addressed biological macromolecules (*e.g.,* nucleic acids or antibodies). The location of each of the macromolecules in the microarray is known, so as to allow for identification of the samples following analysis.

As used herein, the term "array of transcription factor binding targets" refers to an microarray of nucleic acid sequences that are known to, or are suspected of, binding to a transcription factor, arrayed on a solid support.

As used herein, the term "array of transcription factors" refers to an microarray of transcription factor polypeptides on a solid support.

As used herein, the term "SPR surface" refers to a solid surface that is suitable for use in SPR detection. In some embodiments, "SPR surfaces" are "SPR prisms."

As used herein, the term "disposable arrayed SPR prism" refers to a prism that is suitable for use in SPR detection, comprises an arrayed surface (*e.g.,* a microarray), and is not intended to be reused for multiple detection assays. In some embodiments, the disposable arrayed prisms are those disclosed herein.

As used herein, the term "coated on one face" when used in reference to an SPR prism, refers to a prism with a coating on one of the main faces of the prism. For example, in some embodiments, triangular prisms are coated on the upward facing surface. The term "face" is not intended to encompass the small facets on each face of a prism that reflect light.

As used herein, the term "SPR capable metal film" refers to any metallic film that is suitable for use in SPR detection. Examples include, but are not limited to, gold, silver, chrome, and aluminum.

As used herein, the term "microfluidics channels" or "etched microchannels" refers to three-dimensional channels created in material deposited on a solid surface. In some embodiments, microchannels are composed of a polymer (*e.g.,* polydimethylsiloxane). Exemplary methods for constructing microchannels include, but are not limited to, those disclosed herein.

As used herein, the term "one-dimensional line array" refers to parallel microfluidic channels on top of a surface that are oriented in only one dimension.

As used herein, the term "two dimensional arrays" refers to microfluidics channels on top of a surface that are oriented in two dimensions. In some embodiments, channels are oriented in two dimensions that are perpendicular to each other.

As used herein, the term "microchannels" refers to channels etched into a surface. Microchannels may be one-dimensional or two-dimensional.

As used herein, the term "biological macromolecule" refers to large molecules (*e.g.,* polymers) typically found in living organisms. Examples include, but are not limited to, proteins, nucleic acids, lipids, and carbohydrates.

As used herein, the term "target molecule" refers to a molecule in a sample to be detected. Examples of target molecules include, but are not limited to, oligonucleotides (*e.g.* containing a particular DNA binding domain recognition sequence), viruses, polypeptides, antibodies, naturally occurring drugs, synthetic drugs, pollutants, allergens, affector molecules, growth factors, chemokines, cytokines, and lymphokines. As used herein, the term "target nucleic acid sequence" refers to a nucleic acid sequence known to be, or suspected of being, a transcription factor recognition target sequence.

As used herein, the term "binding partners" refers to two molecules (*e.g.,* proteins) that are capable of, or suspected of being capable of, physically interacting with each other. As used herein, the terms "first binding partner" and "second binding partner" refer to two binding partners that are capable of, or suspected of being capable of, physically interacting with each other.

The term "sample" as used herein is used in its broadest sense and includes, but is not limited to, environmental, industrial, and biological samples. Environmental samples include material from the environment such as soil and water. Industrial samples include products or waste generated during a manufacturing process. Biological samples may be animal, including, human, fluid (*e.g.,* blood, plasma and serum), solid (*e.g*., stool), tissue, liquid foods (*e.g*., milk), and cell lysates (*e.g*., cultured cell lysates).

The term "test compound" refers to any chemical entity, pharmaceutical, drug, and the like that is suspected of altering the affinity of a transcription factor for its target sequence. Test compounds comprise both compounds known to alter such interactions, and those suspected to. A test compound can be determined to be active in altering binding interactions by screening using the screening methods of the present invention.

The term "signal" as used herein refers to any detectable effect, such as would be caused or provided by an assay reaction. For example, in some embodiments of the present invention, signals are SPR or fluorescent signals.

As used herein, the term "label free detection" refers to the detection of a binding interaction between unlabeled transcription factors and binding targets. Methods of label free detection include, but are not limited to, those disclosed herein.

As used herein, the term "detection apparatus" refers to an apparatus configured for the detection of an interaction between a transcription factor and a nucleic acid target. In some embodiments, detection apparatus are configured for "label free detection." In other embodiments, they are configured for detection of a label (*e.g.,* on a transcription factor or a DNA binding target).

DNA molecules are said to have "5' ends" and "3' ends" because mononucleotides are reacted to make oligonucleotides or polynucleotides in a manner such that the 5' phosphate of one mononucleotide pentose ring is attached to the 3' oxygen of its neighbor in one direction via a phosphodiester linkage. Therefore, an end of an oligonucleotides or polynucleotide, referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide or polynucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements that direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein, the terms "complementary" or "complementarity" are used in reference to polynucleotides (*i.e.,* a sequence of nucleotides) related by the base-pairing rules. For example, the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands.

The term "homology" refers to a degree of complementarity. There may be partial homology or complete homology (*i.e.,* identity). A partially complementary sequence is one that at least partially inhibits a completely complementary sequence from hybridizing to a target nucleic acid and is referred to using the functional term "substantially homologous." The term "inhibition of binding," when used in reference to nucleic acid binding, refers to inhibition of binding caused by competition of homologous sequences for binding to a target sequence. The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.,* the hybridization) of a completely homologous to a target under conditions of low stringency. This is not to say that conditions of low stringency are such that non-specific binding is permitted; low stringency conditions require that the binding of two sequences to one another be a specific (*i.e.,* selective) interaction. The absence of non-specific binding may be tested by the use of a second target that lacks even a partial degree of complementarity (*e.g.,* less than about 30% identity); in the absence of non-specific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g.,* the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g.,* increasing the temperature of the hybridization and/or wash steps, the use of formamide in the hybridization solution, etc.).

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

When used in reference to a single-stranded nucleic acid sequence, the term "substantially homologous" refers to any probe that can hybridize (*i.e.,* it is the complement of) the single-stranded nucleic acid sequence under conditions of low stringency as described above.

As used herein, the term "hybridization" is used in reference to the pairing of complementary nucleic acids. Hybridization and the strength of hybridization (*i.e.,* the strength of the association between the nucleic acids) is impacted by such factors as the degree of complementary between the nucleic acids, stringency of the conditions involved, the Tₘ of the formed hybrid, and the G:C ratio within the nucleic acids.

As used herein, the term "Tₘ" is used in reference to the "melting temperature." The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41(% G + C), when a nucleic acid is in aqueous solution at 1 M NaC (*See e.g.,* Anderson and Young, Quantitative Filter Hybridization, *in Nucleic Acid Hybridization* [1985]). Other references include more sophisticated computations that take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. Those skilled in the art will recognize that "stringency" conditions may be altered by varying the parameters just described either individually or in concert. With "high stringency" conditions, nucleic acid base pairing will occur only between nucleic acid fragments that have a high frequency of complementary base sequences (*e.g.,* hybridization under "high stringency" conditions may occur between homologs with about 85-100% identity, preferably about 70-100% identity). With medium stringency conditions, nucleic acid base pairing will occur between nucleic acids with an intermediate frequency of complementary base sequences (*e.g.,* hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

"High stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42°C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 ìg/ml denatured salmon sperm DNA followed by washing in a solution comprising 5X SSPE, 0.1% SDS at 42°C when a probe of about 500 nucleotides in length is employed.

As used herein, the term "probe" refers to an oligonucleotide (*i.e.,* a sequence of nucleotides), whether occurring naturally as in a purified restriction digest or produced synthetically, recombinantly or by PCR amplification, that is capable of hybridizing to another oligonucleotide of interest. A probe may be single-stranded or double-stranded. Probes are useful in the detection, identification and isolation of particular nucleic acid sequences.

The term "gene" refers to a nucleic acid (*e.g.,* DNA) sequence that comprises coding sequences necessary for the production of a polypeptide, RNA (*e.g.,* including but not limited to, mRNA, tRNA and rRNA) or precursor (*e.g.,* precursors). The polypeptide, RNA, or precursor can be encoded by a full length coding sequence or by any portion of the coding sequence so long as the desired activity or functional properties (*e.g.,* enzymatic activity, ligand binding, signal transduction, etc.) of the full-length or fragment are retained. The term also encompasses the coding region of a structural gene and the including sequences located adjacent to the coding region on both the 5' and 3' ends for a distance of about 1 kb on either end such that the gene corresponds to the length of the full-length mRNA. The sequences that are located 5' of the coding region and which are present on the mRNA are referred to as 5' untranslated sequences. The sequences that are located 3' or downstream of the coding region and that are present on the mRNA are referred to as 3' untranslated sequences. The term "gene" encompasses both cDNA and genomic forms of a gene. A genomic form or clone of a gene contains the coding region interrupted with noncoding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In particular, the term "gene" refers to the full-length nucleotide sequence. However, it is also intended that the term encompass fragments of the sequence, as well as other domains within the full-length nucleotide sequence. Furthermore, the terms "nucleotide sequence" or " polynucleotide sequence" encompasses DNA, cDNA, and RNA (*e.g*., mRNA) sequences.

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule.

In addition to containing introns, genomic forms of a gene may also include sequences located on both the 5' and 3' end of the sequences that are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers that control or influence the transcription of the gene. The 3' flanking region may contain sequences that direct the termination of transcription, post-transcriptional cleavage and polyadenylation.

As used herein, the term "regulatory element" refers to a genetic element that controls some aspect of the expression of nucleic acid sequences. For example, a promoter is a regulatory element that facilitates the initiation of transcription of an operably linked coding region. Other regulatory elements include splicing signals, polyadenylation signals, termination signals, etc.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

As used herein, the term "reporter gene" refers to a gene encoding a protein that may be assayed. Examples of reporter genes include, but are not limited to, luciferase (*See, e.g.,* deWet *et al*., Mol. Cell. Biol. 7:725 [1987] and U.S. Pat Nos., 6,074,859; 5,976,796; 5,674,713; and 5,618,682; all of which are incorporated herein by reference), green fluorescent protein (*e.g.,* GenBank Accession Number U43284; a number of GFP variants are commercially available from CLONTECH Laboratories, Palo Alto, CA), chloramphenicol acetyltransferase, â-galactosidase, alkaline phosphatase, and horse radish peroxidase.

### DETAILED DESCRIPTION

The present invention provides compositions and methods for the detection of transcription factors binding to their target promoter regions. The present invention further provides methods of screening compounds for their ability to alter such binding interactions. The array-based methods of the present invention are able to overcome many of the disadvantages of previous methods. For example, the array-based method using SPR imaging drastically simplifies the procedure for identifying active transcription factors. The methods of the present invention further provide improved methods of identifying binding target and screening compounds.

### I. Solid Supports

In some preferred embodiments, the present invention utilizes solid supports for performing transcription factor binding assays. The present invention is not limited to a particular solid support. Any number of solid supports may be utilized, including, but not limited to, protein or DNA "chips" composed of any number of suitable materials, and SPR (*e.g.,* metal) surfaces. In some preferred embodiments, solid supports contain arrays of biological macromolecules (*e.g.,* nucleic acids or proteins).

### A. Chips

In some embodiments, the solid support is a "chip." Chips may be made of any suitable material including, but not limited to, metal, plastic, polymer, and glass. Several commercial sources for chips, with and without already arrayed biological molecules, exist (*See e.g.,* the below discussion of arrays). Commercial sources include, but are not limited to, Motorola, Schaumburg, IL; ACLARA BioSciences, Inc., Hayward, CA; Agilent Technologies Inc., Palo Alto, CA; Aviva Biosciences Corp., Dan Diego, CA; Caliper Technologies Corp., Palo Alto, CA; Clontech, Palo Alto, CA; Corning, Acton, MA; Gene Logic Inc., Columbia, MD; Hyseq Inc., Sunnyvale, CA; Incyte Genomics, Palo Alto, CA; Micronics Inc., Redmond, WA; Mosaic Technologies, Waltham, MA; OriGene Technologies, Rockville, MD; Packard Instrument Corp., Meriden, CT; Rosetta Inpharmatics, Kirkland, WA; and Sequenom, San Diego, CA.

### B. SPR Surfaces

In other embodiments, the solid support is an SPR surface. Surface Plasmon Resonance techniques involve a surface coated with a thin film of a conductive metal, such as gold, silver, chrome or aluminum, in which electromagnetic waves, called Surface Plasmons, can be induced by a beam of light incident on the metal glass interface at a specific angle called the Surface Plasmon Resonance angle. Modulation of the refractive index of the interfacial region between the solution and the metal surface following binding of the captured macromolecules causes a change in the SPR angle which can either be measured directly or which causes the amount of light reflected from the underside of the metal surface to change. Such changes can be directly related to the mass and other optical properties of the molecules binding to the SPR device surface. Several biosensor systems based on such principles have been disclosed (*See e.g.,* WO 90/05305).

Generally, in a Kretschman-configuration SPR device, a glass cover slip or slide of appropriate refractive index is coated with a thin (on the order of 50 nm) SPR-capable metal layer. This metal surface is then chemically patterned, and probe molecules are attached to the pattern features. The patterning can be either a basic grid-like array, or microfluidic channels can be overlaid onto the surface for probe deposition and sample application. This gold coated, patterned slide is then optically linked to a prism. This linkage is accomplished by placing a thin film of index-matching fluid between the prism and the slide. A sample solution is then passed over the probes arrayed on the surface. Interaction of an analyte in the solution with a probe molecule on the surface is detected as a change in refractive index. Importantly, SPR detection is label-free.

In some embodiments, a disposable SPR prism is utilized. The prism may be made of any suitable material including, but not limited to, glass and silica. In preferred embodiments, prisms are made of a high refractive index material. Preferred materials are those whose SPR minimum falls within an angle range. The range can be determined by applying known formulas (*See e.g.,* Hansen, W. N. Journal of the Optical Society of America 53(3):380-390). For example, in some embodiments, prisms are made from a material including, but not limited to, BK-7 glass, SFL-6 glass, and preferably SF-10 glass.

In some embodiments, the prisms are coated on one face with an SPR-capable metal layer. The present invention is not limited to a particular type of metal. Any metal that is suitable for use in SPR may be utilized including, but not limited to, gold, silver, chrome or aluminum. The thickness of the metal film is not overly critical insofar as the film is uniformly applied and will function in SPR imaging analysis. In preferred embodiments, a film of about 450 Å thick is used. In preferred embodiments, gold is utilized as the SPR capable film to coat the prisms.

In some embodiments, the metal (*e.g.,* gold) layer is chemically patterned for attachment of molecular probes (*e.g.,* biomolecules). The present invention is not limited to a particular biological macromolecule. A variety of biological macromolecules are contemplated including, but not limited to, DNA, proteins, carbohydrates, lipids and amino acids.

### C. Arrays

In some embodiments, solid surfaces are chemically patterned for attachment of biological macromolecules (e.g., nucleic acids or proteins). In some embodiments, the present invention further provides solid supports comprising arrays of biological macromolecules. In preferred embodiments, arrays comprise at least 50, preferably at least 100, even more preferably at least 1000, still more preferably, at least 10,000, and yet more preferably, at least 100,000 distinct biological macromolecules. In preferred embodiments, each distinct biological macromolecule is addressed to a specific location on the array. In preferred embodiments, each addressable location is larger than 25, and preferably, larger than 50 microns.

The present invention is not limited to a particular method of fabricating or type of array. Any number of suitable chemistries known to one skilled in the art may be utilized.

### 1. Amine Modified Surface Arrays

In some preferred embodiments, the method of generating arrays described in U.S. Patent 6,127,129 (herein incorporated by reference) is utilized. In the first step of the method, a monolayer of a thiol is self-assembled from an ethanolic solution onto a solid support, which has been coated with a thin noble-metal film. The present invention is not limited to a particular thiol. A variety of lengths and positions of attachment of the thiol group are contemplated as being suitable for use in the present invention. In some preferred embodiments, long chain (*e.g.,* 11 carbon) alkanethiols are utilized. In other embodiments, branched or cyclic thiols are utilized.

In some embodiments, amine (*e.g.,* MUAM) or carboxylic acid terminated *(e.g.,* MUA), hydroxyl terminated (*e.g.,* MUD), or MUAM modified to be thiol terminated are utilized. In some particularly preferred embodiments, an ù-modified alkanethiol, preferably an amine-terminated alkanethiol, most preferably 11-mercaptoundecylamine (MUAM), is utilized (*See e.g.,* Thomas *et al*., J Am. Chem. Soc. 117:3830 [1995]).

Self-assembled monolayers of ù-modified alkanethiols on gold form well ordered, monomolecular films. However, if left exposed for extended periods of time, the terminal amine groups of amino-modified alkanthiols may react with CO₂ to form carbamate salts on the surface. Consequently, it is preferred that exposure of amino-terminated alkanethiol-coated substrates to CO₂ be minimized.

Next, the alkanethiol-covered surface is reacted with a reversible protecting group to create a hydrophobic surface. In certain embodiments utilizing an amine-modified alkanethiol such as MUAM, the protecting group is an amino protecting group, preferably 9-fluorenylmethoxycarbonyl (Fmoc). The present invention is not limited to an Fmoc protecting group. Any reversible protecting group may be utilized. Preferred protecting groups offer efficient protection, favorable (*e.g.,* to biological molecules) deprotecting conditions, efficient deprotection, and are suitable for reactions on a surface. For example, in some embodiments, Tboc is utilized for the protection of alkanethiols.

Fmoc is a bulky, hydrophobic, base labile, amine protecting group routinely used in the solid phase synthesis of peptides. The choice of protecting group used is dependent in large measure upon the nature of the ù-modification made to the alkanethiol. If the ù-modification is the addition of a carboxyl group, a hydrophobic carboxy protecting group is preferred. Likewise, if the ù-modification is the addition of a hydroxyl or thiol group, a hydrophobic hydroxy or thiol protecting group, respectively, is preferred used. Any type of hydrophobic protecting group suitable for protecting the ù -modification used on the alkanethiol can be utilized in the present invention. Numerous such protecting groups, for any number of reactive moieties, such as amine, hydroxy, ester, carbamate, amides, ethers, thoioethers, thioesters, acetals, ketals and carboxy functionalities, are known to the art *(See e.g.,* Frutos *et al*., Langmuir 16:2192 [2000]). For example, chloride derivatives of both Fmoc and trityl can be used to reversibly modify hydroxyl-terminated alkanethiols.

In some embodiments utilizing Fmoc protecting groups, the N-hydroxysuccinimide ester of Fmoc (Fmoc-NHS) is reacted with the terminal amine moiety of the MUAM molecule to form a stable carbamate (urethane) linkage, covalently attaching the Fmoc group to the surface.

Subsequently, the bond anchoring the alkanethiol to the metal substrate is selectively cleaved to yield a patterned surface of exposed metal. In some preferred embodiments, UV photopatterning is utilized to create the patterned surface. However, any suitable method of generating a patterned surface may be utilized. For example, in some embodiments, microcontact printing methods can also be used to yield a patterned surface. Using UV patterning, the surface is exposed through a quartz mask to UV radiation, which photo-oxidizes the gold-sulfur bond that anchors the alkanethiol monolayers to the surface. The surface is then rinsed, removing the photo-oxidized alkanethiol and leaving an array of bare metal pads surrounded by a hydrophobic MUAM+Fmoc background. Using photopatterning, features with dimensions as small as 50 mm have been achieved; using microcontact printing methods, arrays with features as small as about 100 nm are achievable.

The surface is next exposed to an alkanethiol solution (in some preferred embodiments, an ethanolic solution of MUAM) whereby the alkanethiol assembles into the bare gold regions producing a surface composed of hydrophilic alkanethiol pads surrounded by the hydrophobic blocked background. This difference in hydrophobicity between the reactive alkanethiol regions and the background is useful for the pinning of small volumes of aqueous biomolecule or cell solutions onto individual array locations.

Biological macromolecules are then covalently attached to the surface. The alkanethiol active pads are first exposed to a solution of a bifunctional linker. Preferred linkers are those capable of binding at one end to the alkanethiol surface and at the other end to the biological macromolecule to be immobilized to form the desired array. Any bifunctional (*e.g.,* hetero or homo bifunctional) linker having these characteristics can be used in the present invention (*See e.g.,* Smith *et al*., Langmuir 17:2502 [2001] and the Catalog of Pierce Chemical Company, Rockford, IL). Exemplary linkers include, but are not limited to, SSMCC, DSS, and PDITC.

The preferred bifunctional linker is sulfosuccinimidyl 4-(N-maleimidomethyl)cyclohexane-1-carboxylate (SSMCC), a heterobifunctional linker which contains both an N-hydroxysulfosuccinimide (NHSS) ester and a maleimide functionality. The NHSS ester end of the molecule reacts with the free amine groups on an amino-modified surface, such as the MUAM spots, creating pads terminated in maleimide groups, which are reactive towards thiols. Small volumes (0.08 to 0.1 L) of 1 mM solutions of 5'-thiol-modified biological macromolecules (e*.g.,* DNA sequences) are then spotted at discrete array locations and react to form a covalent attachment to the surface. Using this technique, any number of biological macromolecules can be spotted at different array locations.

The protecting group (*e.g.,* Fmoc) is next removed from the array surface. Preferably, this is accomplished by exposure to a 1M solution of the secondary amine, TAEA, in DMF. Many basic secondary amines can be used to remove Fmoc from the surface (*e.g.,* including, but not limited to, 1M solutions of ethanolamine and piperidine). After the deprotection step, the array background has been converted back to the original alkanethiol surface.

In the final step of the array fabrication, the alkanethiol background is reacted with a compound to create a background that is resistant to the non-specific binding of proteins. The preferred compound for this purpose is PEG-NHS, although any compound that will selectively bind to the alkanethiol surface and inhibit non-selective protein binding can be used. In order to effectively monitor the binding of proteins to arrays of surface-bound biomolecules or cells, it is preferred that the array background prohibit the non-specific adsorption of protein molecules. Additional blocking groups include, but are not limited to, mixtures of PEG-terminated and other molecules (*e.g.,* hydroxyl-terminated), different molecular weights of PEG molecules, polylysine, casein, BSA, and octadecane thiol (*See e.g*., Chapman *et al*., J. Am. Chem. Soc., 122:8303 [2000]).

### 2. Additional Arrays

The present invention is not limited to the array fabrication methods described above. Additional array generating technologies may be utilized, including, but not limited to, those described below.

In some embodiments, a DNA array is generated using photolithography on a solid surface (Affymetrix, Santa Clara, CA; *See e.g.,* U.S. Patent Nos. 6,045,996; 5,925,525; and 5,858,659; each of which is herein incorporated by reference). The technology uses miniaturized, high-density arrays of oligonucleotide probes affixed to the solid surface. Probe arrays are manufactured by Affymetrix's light-directed chemical synthesis process, which combines solid-phase chemical synthesis with photolithographic fabrication techniques employed in the semiconductor industry. Using a series of photolithographic masks to define exposure sites, followed by specific chemical synthesis steps, the process constructs high-density arrays of oligonucleotides, with each probe in a predefined position in the array.

In other embodiments, a DNA array containing electronically captured probes (labeled nucleic acid sequences) (Nanogen, San Diego, CA) is utilized (*See e.g.,* U.S. Patent Nos. 6,017,696; 6,068,818; and 6,051,380; each of which are herein incorporated by reference). In some embodiments, a modified method of Nanogen's technology, which enables the active movement and concentration of charged molecules to and from designated test sites on a semiconductor microchip is utilized. DNA capture probes are electronically placed at, or "addressed" to, specific sites on the solid support. Since DNA has a strong negative charge, it can be electronically moved to an area of positive charge.

First, a test site or a row of test sites on the solid support is electronically activated with a positive charge. Next, a solution containing the DNA probes is introduced onto the solid support. The negatively charged probes rapidly move to the positively charged sites, where they concentrate and are chemically bound to a site on the solid support. The solid support is then washed and another solution of distinct DNA probes is added until the array of specifically bound DNA probes is complete.

In still further embodiments, an array technology based upon the segregation of fluids on a flat surface (chip) by differences in surface tension (ProtoGene, Palo Alto, CA) is utilized (*See e.g.,* U.S. Patent Nos. 6,001,311; 5,985,551; and 5,474,796; each of which is herein incorporated by reference). Protogene's technology is based on the fact that fluids can be segregated on a flat surface by differences in surface tension that have been imparted by chemical coatings. Once so segregated, oligonucleotide probes are synthesized directly on the surface by ink-jet printing of reagents. The array with its reaction sites defined by surface tension is mounted on a X/Y translation stage under a set of four piezoelectric nozzles, one for each of the four standard DNA bases. The translation stage moves along each of the rows of the array and the appropriate reagent is delivered to each of the reaction site. For example, the A amidite is delivered only to the sites where amidite A is to be coupled during that synthesis step and so on. Common reagents and washes are delivered by flooding the entire surface and removing by spinning. DNA probes unique for the target sequence of interest are affixed to the solid support using Protogene's technology. The prism is then contacted with a test sample of interest. Following hybridization, unbound DNA is removed and hybridization is detected using SPR.

### 3. Microfluidics

In some embodiments, arrays are fabricated by patterning the solid support with microfluidic channels. In some embodiments, microfluidics are generated using the polydimethylsiloxane (PDMS) polymer-based methods described by Lee *et al*. (Analytical Chemistry, 73:5525 [2001]). This technique can be used for both fabricating 1-D DNA microarrays using parallel microfluidic channels on chemically modified gold and silicon surfaces, and in a microliter detection volume methodology that uses 2-D DNA microarrays formed by employing the 1-D DNA microarrays in conjunction with a second set of parallel microfluidic channels for solution delivery.

For example, in some embodiments, microliter detection volume methodology that uses 2-D DNA hybridization microarrays formed by employing 1-D DNA line arrays in conjunction with a second set of parallel microfluidic channels for solution delivery is utilized. In some embodiments, PDMS microchannels are fabricated by replication from 3-D silicon wafer masters that were created photolithographically from 2-D chrome mask patterns (*See e.g.,* Duffy *et al*., Anal. Chem., 70:4974 [1998] and Effenhauser *et al*., Anal. Chem., 69:3451 [1997]).

A gold thin film surface deposited on the solid support is reacted with MUAM in order to form a self-assembled monolayer on the gold surface as described above. A PDMS polymer film containing parallel microchannels is then attached to the MUAM modified gold surface. In some embodiments, a surface pattern is created by flowing the heterobifunctional linker SSMCC through the PDMS microchannels over the gold surface. The SSMCC reacts with the MUAM to create a maleimide-terminated alkanethiol monolayer. Biological macromolecules (*e.g*., 5'-thiol-modified DNA or RNA probes) are then each flowed into a separate PDMS microchannel and react with the maleimide-terminated gold surface to form an array of probes on the surface of the gold. In some embodiments, the microchannels are cleaned with water, the PDMS is removed from the surface and the gold slide is soaked in a PEG-NHS solution in order to modify the MUAM background (see above description of blocking with PEG-NHS). The PEG-coated background helps to eliminate nonspecific adsorption of DNA or RNA during hybridization experiments.

The present invention is not limited to a particular method of fabricating channels in the solid surfaces of the present invention. For example, in other embodiments, the present invention utilizes microchannels etched into the surface (*See e.g.,* U.S. Patent 6,176,962, herein incorporated by reference). In still further embodiments, microfluidic channels are fabricated using wet chemical etching (Wang *et al*., Anal. Chem., 72:2514 [2000]) or soft lithography (Deng *et al*., Anal. Chem. 72:3176 [2000]).

### 4. Array Processing

In some embodiments, following patterning or generation of arrays, a silicone gasket (Grace Biolabs, Bend, Or) is sandwiched in-between the solid surface and a microscope cover slide to form a small reaction chamber. In other embodiments, a HYBRIWELL seal (Grace Biolabs) is used to create a low-volume reaction chamber.

### II. Transcription Factor Binding Reactions on a Solid Support

The present invention provides methods and compositions for the detection of transcription factor binding to target nucleic acid sequences. The below description provides several exemplary methods.

### A. Target Nucleic Acids

In some embodiments, target nucleic acid sequences are attached to surfaces configured for label-free (*e.g.,* SPR) detection. In preferred embodiments, targets are oligonucleotides. In some particularly preferred embodiments, oligonucleotide targets are double stranded.

In some preferred embodiments, arrays of target nucleic acid sequences are attached to the solid surfaces. In some embodiments, multiple copies of the same transcription factor target sequence are attached to different places on the array. In other embodiments, different target sequences are attached to each place on the array. In some embodiments, the target sequence is a known sequence. In still further embodiments, randomized target sequences are attached to arrayed surfaces.

### B. Transcription Factors

The present invention is not limited to the analysis of a particular transcription factor. Transcription factors from any organism or cell type may be utilized. In some embodiments, transcription factors are known proteins with known binding targets. In other embodiments, transcription factors are known proteins but their particular target sequences are unknown. In still further embodiments, mutants or variants of known transcription factors are utilized. In yet other embodiments, unknown transcription factors or cell cultures containing such transcription factors are utilized to screen known target sequences.

In some embodiments, transcription factors are attached to solid surfaces (*e.g.,* as arrays of transcription factors). In some embodiments, multiple copies of the same transcription factor are attached as arrays on solid surfaces. In other embodiments, different transcription factors are attached to each location of an array on a solid surface.

### C. Binding Assays

In some embodiments, purified transcription factors or crude protein mixtures (*e.g.,* cell nuclear extracts) are exposed to an array of double stranded oligonucleotide probes on the surface of a solid support. In some embodiments, the same DNA sequence is applied at multiple addresses in the array. In some embodiments, each probe is tested by directly spotting experimental solutions on the probe. Alternatively, in other embodiments, each probe is tested by using microfluidics to deliver the sample to individual probe spots. In other embodiments, different target sequences are arrayed and on or more candidate transcription factors are assessed for their ability to interact with the target sequences. In still further embodiments, cells or cell lysates comprising transcription factors are exposed to a variety of growth conditions or test compounds prior to contacting the transcription factors with the arrays of DNA targets. The binding affinity is compared in the presence of various growth conditions or test compounds to the binding in the absence. Binding of transcription factors is detected using any suitable method (*e.g.,* those described below).

In other embodiments, arrays of transcription factors are exposed to nucleic acid target sequences in solution. In some embodiments, microfluidics are utilized to deliver the nucleic acids to the individual spots on the array. In some embodiments, transcription factors are exposed to test compounds prior to being exposed to nucleic acid targets. In some embodiments, microfluidics are used to expose some arrayed transcription factors, but not others, to the test compounds. The binding affinity is compared in the presence of various test compounds to the binding in the absence. Binding of transcription factors is detected using any suitable method (*e.g.,* those described below).

### D. Detection Methods

The binding of a transcription factor to a nucleic acid target may be detected using any suitable method, including but not limited to, label free detection, detection of a label, or a combination method. In some embodiments, binding is quantitated.

### 1. Label Free Detection

In some preferred embodiments, detection is label free. For example, in some embodiments, SPR (*See e.g.,* above description) is utilized. In other embodiments, the label free electrical detection method described in WO 01/61053A2 (herein incorporated by reference) is utilized.

In still further embodiments, oligonucleotide-conjugated nanoparticles are utilized for detection of binding (*See e.g.,* Nanosphere, Northbrook, IL, U.S. Patent 6,361,944, herein incorporated by reference). The assay involves a detectable change (*e.g.,* a color change, the formation of aggregates of the nanoparticles, or the precipitation of the aggregated nanoparticles) that occurs upon hybridization of the oligonucleotides on the nanoparticles to the nucleic acid. The color changes can be observed with the naked eye or spectroscopically. The formation of aggregates of the nanoparticles can be observed by electron microscopy or by nephelometry. The precipitation of the aggregated nanoparticles can be observed with the naked eye or microscopically.

In some embodiments, oligonucleotide arrays are labeled with nanoparticles and detected using the described methods. In other embodiments, following binding of a transcription factor to a bound target, nucleic acid sequences not bound to a target are detected by oligonucleotide-nanoparticle conjugates (*e.g.,* the absence of a signal is indicative of a positive binding event). In still further embodiments where a transcription factor is bound to the solid support, bound nucleic acids are detected using the conjugates (*e.g.,* by an oligonucleotide-nanoparticle that is complementary to the target nucleic acid).

### 2. Additional Detection Methods

In some embodiments, detection via a label is utilized. In some embodiments, detection via a label is combined with label free (*e.g.,* SPR) detection methods. In other embodiments, detection with a label is utilized independent of label-free detection.

Detection thresholds are often a limiting factor in SPR detection methods. SPR detection is primarily a function of changes in mass adherent to the SPR surface. Methods that greatly increase or decrease the bound mass affect sensitivity. Thus, in some embodiments, addition detection methods are utilized to alter the mass of the complex being detected.

For example, in some embodiments, transcription factors or nuclear extracts are mixed with target nucleic acid sequences in a vessel. The target nucleic acids are allowed to bind the transcription factors. Unbound nucleic acids are separated from bound nucleic acids. Bound complexes are then detected by hybridization to a second nucleic acid arrayed on an SPR-capable surface. In some embodiments, the first nucleic acid sequence has a 'sticky end' that protrudes from the transcription factor binding site. This sticky end hybridizes to the complementary second nucleic acid on the array surface. The bound mass is therefore increased by the mass of the primary DNA probe. In some embodiments, the mass is further increased by conjugation of the target nucleic acid to a label or nanoparticles (*e.g.,* gold).

In other embodiments, antibodies are utilized for enhancing the SPR signal generated by transcription factor-target nucleic acid sequence complexes. The transcription factor directly binds to the arrayed target DNA. In some embodiments, the SPR signal is then enhanced by binding of an antibody to the transcription factor. In some embodiments, the antibody is labeled (*e.g.,* with fluorescent labels (*e.g.,* fluorescein), enzymatic detection labels (*e.g.,* horse radish peroxidase), and metal labels (*e.g.,* gold)). This method has the further advantage of immunologically confirming the identity of the protein binding to the DNA.

### 3. Quantitation

In some preferred embodiments, binding assays are quantitative. For example, the detection methods of the present invention allow for investigation of the kinetics of binding. Kinetic measurements are obtained by taking multiple time points and analyzing the rate of increase in signal.

### III. Applications of Binding Assays

The binding assay so the present invention find use in a variety of applications, including, but not limited to, the determination of binding sequences, screening of growth conditions, and screening of test compounds.

### A. Determination of Binding Sequences

In some embodiments, the present invention provides methods of determining the target nucleic acid binding sequence of transcription factors. Because the DNA sequence bound by a protein cannot be accurately predicted from its primary sequence, it is very hard to determine the sequence-specific binding site of transcription factor homologs, mutants, and variants. Knowledge of the binding site is essential for determining the genes regulated by a given transcription factor. Accordingly, in some embodiments of the present invention, transcription factor recognition sequences are identified by exposing the purified protein to an array of double stranded DNA molecules and measuring protein binding. In some embodiments, degenerate nucleic acid sequences are generated based on suspected binding sequences. In other embodiments, random DNA sequences are utilized. In preferred embodiments, arrays are addressed such that sites giving a positive binding signal can be identified.

In some embodiments, label-free *(e.g.,* SPR) imaging is used to measure binding to thousands of arrayed sequences simultaneously. Thus, the present invention provides methods of identifying binding sites for cloned transcription factor variants without any prior knowledge of the recognition site.

Many proteins bind DNA non-specifically. Specificity for the identified binding site can be confirmed using any suitable method. For example, in some embodiments, binding kinetics (*e.g.,* as measured by SPR) gives an indication of binding specificity. In other embodiments, competitor DNA of identical or nearly identical sequence is used to compete off specifically bound transcription factors. SPR is preferred for making such measurements, since none of the molecules (*e.g.,* transcription factor, target DNA or competitor DNA) need be labeled. Furthermore, transcription factors can bind to related, but degenerate, target DNA sequences with various affinities. This often has significant impact on gene regulation. In some embodiments of the present invention, binding affinity for related DNA sequences is measured on a DNA array by SPR. Both the binding kinetics and the amount of transcription factor bound to a particular sequence can be determined.

### B. Screening for Growth Conditions

In other embodiments, the methods of the present invention are utilized to measure binding of transcription factors from cell extracts. Such information is useful to researchers studying regulation of particular genes. In some preferred embodiments, cells, tissues or organisms are exposed to two different conditions (*e.g.,* media, temperature, antibiotics, etc.) and differences in binding resulting from this treatment can be observed. The methods of the present invention provide the added advantage that whole suites of regulatory sequences can be screened for binding simultaneously. This knowledge is essential for the understanding of regulatory networks in cells. In other embodiments, the methods of the present invention are used to identify previously unknown sequence-specific DNA binding functions induced by the test treatment by exposing the whole array and observing novel binding. In some embodiments, sensitivity and cell extract volume requirements are enhanced by using microfluidics or targeted spotting of array features to apply the cell extract to particular surface addresses.

### C. Screening Test Compounds

In still further embodiments, the methods of the present invention are used to measure the effect of small molecule test compounds (*e.g.,* including, but not limited to, drugs (*e.g*., cancer drugs), suspected carcinogens, antibiotics, growth conditions, and cell type) on the DNA binding activity of purified transcription factors. For example, in some embodiments, a cell extract is prepared and divided into two aliquots. The test compound is added to one aliquot, but not the other, and purified transcription factor is mixed with both. The change in state of the transcription factor (bound vs. unbound or the affinity of binding) is then measured by observing transcription factor binding to the arrayed DNA. The effect of the small molecule on activation or inactivation of multiple transcription factors is assayed simultaneously using such arrays.

In other embodiments, the effect of the small molecule on transcription factor binding is assayed directly without the use of cell extracts. This is useful for transcription factors that can be switched on or off using simple in vitro reactions, such as phosphorylation.

In still further embodiments, arrays of transcription factors are exposed to test compounds. In some embodiments, test compounds are removed from the array prior to testing. In other embodiments, test compounds are left on the array during testing. The array is then contacted with target nucleic acid sequences and the binding measured.

### EXPERIMENTAL

The following examples are provided in order to demonstrate and further illustrate certain preferred embodiments and aspects of the present invention and are not to be construed as limiting the scope thereof.

### Example

### Detection of AP2 transcription factor binding to arrayed probes

### Materials Oligonucleotide sequences:

AP2 immobilized oligo
5' thiol TTT TTT TTT TTT TTT  GAT CGA ACT GAC CGC CCG CGG CCC GT 3'
(SEQ ID NO:1)

### Complement to the AP2 oligonucleotide

5' ACG GGC CGC GGG CGG TCA GTT CGA TC 3' (SEQ ID NO:2)

The AP2 transcription factor protein was purchased as part of the Promega Core Footprinting System (Promega, Madison, WI USA). The buffer used during AP2 protein-DNA binding reactions (binding buffer) was also obtained as part of the Promega Core Footprinting System. The oligonucleotides were hybridized in 20 mM phosphate, 250mM NaCl, pH 7.78 hybridization buffer.

### Surface preparation:

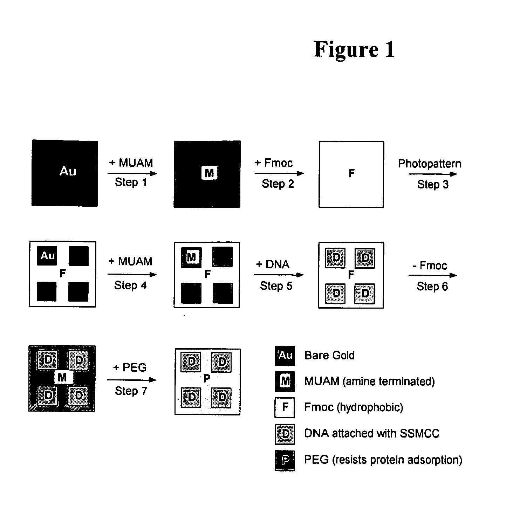

A photopatterned MUAM surface was created on an SPR-capable gold coated glass slide as described above and outlined in Figure 1. Briefly, a MUAM monolayer was deposited on the thin gold film from an ethanolic MUAM solution. The amine surface was then blocked with the protecting group Fmoc, and selected areas of Fmoc-MUAM were removed by photopatterning with UV light and a quartz mask. The Fmoc-MUAM was cleaved from the surface in the areas exposed to UV, regenerating bare gold squares surrounded by Fmoc-MUAM. Fresh MUAM was deposited in the squares, and reacted with the heterobifunctional crosslinker SSMCC, thereby creating thiol-reactive maleimide-MUAM squares surrounded by fmoc-MUAM. The surface was rinsed to remove unreacted SSMCC with distilled water. Thiol modified AP2 consensus oligo nucleotide, at 1 mM in SSPE buffer, was spotted onto the SSMCC squares using a picopump. The surface was allowed to sit overnight at room temperature in a humid chamber to affect binding of the thiol to the maleimide (SSMCC) surface.

The slides were then deprotected by exposure to a 1 M solution of the secondary amine, TAEA, in DMF to remove the fmoc. PEG-NHS (MW 2000) was added to the deprotected background regions to limit non-specific protein binding. The slides were placed in a GWC Instruments (Madison, WI) SPR imager, and hybridization buffer was flowed over the surface for approximately five minutes to equilibrate the surface. A background image was captured using the SPR imager software for use as a background mask. The background mask image is used to calculate difference images used to detect later binding events on the surface. Complement DNA was flowed in at a concentration of 2 micromolar in hybridization buffer. Hybridization was detected as an increase in real-time SPR signal in difference images on the regions modified with the thiol AP2 DNA. Minimal increase in background signal was observed in the background regions lacking AP2 DNA. This created the double-stranded DNA sequences necessary to act as AP2 protein binding targets.

### Results:

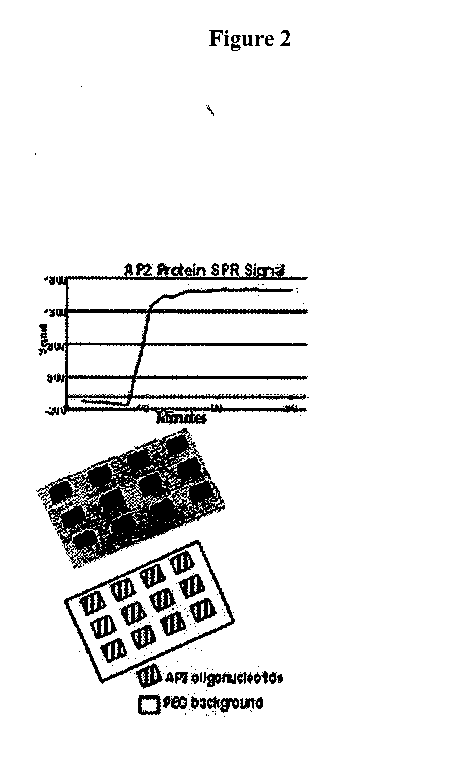

For AP2 binding, the slides were flushed with 50 µL binding buffer diluted into 450 µL of hybridization buffer. This solution was flowed across the surfaces for several minutes to equilibrate the system. A new background mask image was captured at this point for use in calculating protein binding difference images, and image collection begun. After 5 minutes, 5 µL (8.75pmol) of AP2 extract was added. The buffer was then exchanged for hybridization buffer, and the surface was equilibrated to the background mask image conditions. Higher signal was generated at the regions where double stranded AP2 consensus oligos were bound as compared to background regions, indicating protein binding to the double-stranded oligos. This data is shown in Figure 2. Figure 2 top is a graph of the real-time change in SPR signal measured on AP2 DNA target spots after addition of AP2 protein. Figure 2 middle shows the final difference image after AP2 binding to the arrayed AP2 oligonucleotides. Figure 2 bottom is a schematic representation of the array surface used in these experiments.

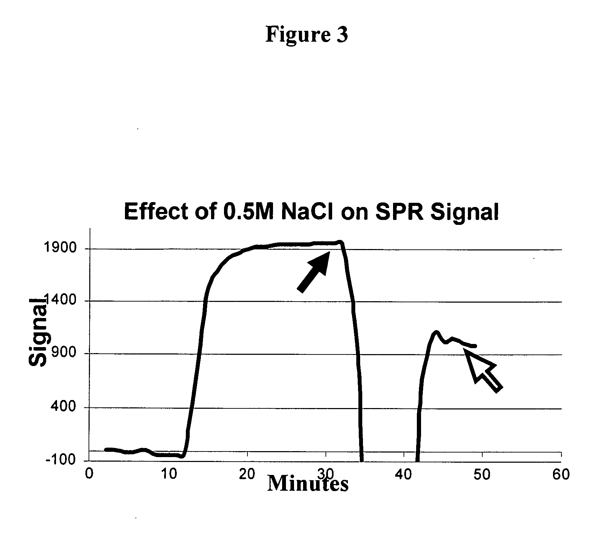

A second experiment examined the effect of high salt on AP2 binding. An identical surface was hybridized with complement then AP2 protein extract was added as described above. Binding occurred as before. 45 µL of 3 M salt was added to the AP2 binding buffer in hybridization buffer solution. This increased the NaCl concentration from 250 to500 mM. This solution was washed over the surface during the imaging for about 5 minutes then replaced with fresh binding buffer in hybridization buffer. Upon rinsing with the fresh binding buffer in hybridization buffer, the generated SPR signal was approximately half of the original signal (Figure 3). This suggests that AP2 protein binding is reduced when exposed to higher salt concentration conditions.

These results demonstrate that an array format can be used to observe transcription factor binding to arrayed double-stranded DNA targets. The surface and detection method were chosen as examples; those knowledgeable in the art will appreciate that several surfaces and detection methods, including but not limited to those described herein, can be used to perform similar measurements.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention that are obvious to those skilled in the relevant fields are intended to be within the scope of the following claims.

## Claims

1. A composition comprising an arrayed solid surface, said solid surface comprising an array of transcription factor binding targets.

2. The composition of claim 1, wherein said array of transcription factor binding targets comprises at least 20, preferably at least 50, preferably at least 100, more preferred at least 1000 distinct target nucleic acid sequences.

3. The composition of claim 1, wherein said transcription factor targets are double-stranded DNA molecules.

4. A composition comprising an arrayed solid surface comprising an array of transcription factors.

5. The composition of claim 4, wherein said array of transcription factors comprises at least 20, preferably at least 50, preferably at least 100, more preferred at least 1000 distinct transcription factors.

6. The composition of claim 1 or 4, wherein said solid surface is configured for label free detection.

7. The composition of claim 6, wherein said solid surface is an SPR surface.

8. The composition of claim 7, wherein said SPR surface is an SPR prism.

9. The composition of claim 1 or 4, wherein said solid surface further comprises a plurality of microfluidics channels.

10. The composition of claim 9, wherein said microfluidics channels are one-dimensional line arrays.

11. The composition of claim 9, wherein said microfluidics channels are two-dimensional arrays.

12. The composition of claim 1 or 4, wherein said solid surface further comprises a plurality of etched microchannels.

13. A composition according to claim 4 in contact with a sample comprising at least one transcription factor binding target.

14. A composition according to claim 4 in contact with a treated biological sample, said treated biological sample used to prepare a cell lysate containing at least one test compound.

15. A system, comprising a composition according to claim 1 or 2; at least one transcription factor polypeptide; and a detection apparatus in communication with said arrayed solid surface.

16. The system of claim 15, wherein said at least transcription factor polypeptide comprises at least 20, preferably at least 50, preferably at least 100, more preferred at least 1000 distinct transcription factor polypeptides.

17. The system of claim 15, further comprising competitor DNA, wherein said competitor DNA has an identical nucleic acid sequence as said transcription factor binding targets.

18. The system of claim 15, further comprising an antibody that specifically recognizes said at least on transcription factor polypeptide.

19. The system of claim 18, wherein said antibody is conjugated to a gold particle.

20. The system of claim 15, further comprising a second transcription factor target sequence, wherein a portion of said second transcription factor target sequence is complementary to said arrayed transcription factor target sequences.

21. The system of claim 15, further comprising at least one test compound.

22. The system of claim 21, wherein said at least one test compound is a drug.

23. The system of claim 15, wherein said solid surface is an SPR surface.

24. The system of claim 23, wherein said SPR surface is an SPR prism.

25. The system of claim 15, wherein said solid surface further comprises a plurality of microfluidics channels.

26. The system of claim 25, wherein said microfluidics channels are one-dimensional line arrays.

27. The system of claim 25, wherein said microfluidics channels are two-dimensional arrays.

28. The system of claim 15, wherein said detection apparatus is configured for label-free detection.

29. The system of claim 15, wherein said detection apparatus is configured for detection of a label.

30. A system, comprising a composition according to claim 4; at least one transcription factor binding target; and a detection apparatus in communication with said arrayed solid surface.

31. A composition according to claim 1 in contact with a biological sample containing at least one transcription factor.

32. A composition according to claim 31, wherein said biological sample has been treated with a small molecule.

33. A system, comprising a composition according to claim 4; at least one transcription factor binding target; and a cell lysate comprising at least one test compound, and said cell lysate in contact with said array solid surface.

34. A method of detecting biomolecular interactions, comprising:
a) providing a system according to any of the claims 15 to 29;
b) contacting said at least one transcription factor polypeptide with said array of transcription factor targets under conditions such that said apparatus detects interactions between said array of transcription factor targets and said at least one transcription factor polypeptide.

35. The method of claim 34, further comprising the step of contacting said at least one transcription factor polypeptide and said array of transcription factor targets with competitor DNA, wherein said competitor DNA has an identical nucleic acid sequence as said transcription factor binding targets.

36. The method of claim 34, further comprising providing an antibody that specifically recognizes said at least on transcription factor polypeptide.

37. The method of claim 36, further comprising the step of detecting said interaction between said transcription factor polypeptide and said transcription factor binding target by detecting the binding of said antibody to transcription factor polypeptide bound to said transcription factor binding target.

38. The method of claim 36, wherein said antibody is conjugated to a gold particle.

39. The method of claim 34, further comprising providing a second transcription factor target sequence, wherein a portion of said second transcription factor target sequence is complementary to said arrayed transcription factor target sequences.

40. The method of claim 39, further comprising, prior to said contacting, the step of contacting said second transcription factor target with said transcription factor polypeptide under conditions such that said second transcription factor target and said transcription factor polypeptide interact.

41. A method of detecting biomolecular interactions, comprising:
a) providing
i) a composition according to claim 4;
ii) at least one transcription factor binding target; and
iii) a detection apparatus in communication with said arrayed solid surface; and
b) contacting said at least one transcription factor binding target with said array of transcription factors under conditions such that said apparatus detects interactions between said array of transcription factors and said at least one transcription factor binding target.

42. A method for measuring the effect of small molecules on biomolecular interactions, the method comprising:
a) providing
i) a composition according to claim 1;
ii) a test compound;
iii) a biological sample comprising at least one transcription factor;
iv) a detection apparatus; and
b) treating said biological sample with said small molecule;
contacting said treated biological sample with said array of transcription factor targets under conditions such that said apparatus detects interactions between said array of transcription factor targets and said at least one transcription factor contained in said biological sample.

43. The method of claim 42, wherein, prior to said step of contacting treated biological sample with said array of transcription factor, said biological sample is treated with a test compound.

44. The method of claim 43, wherein said test compound is a drug.

45. The method of claim 42, further comprising the step of comparing said interactions between said array of transcription factor targets and said at least one transcription factor polypeptide in the presence of said test compound to said interactions in the absence of said test compound.

46. The method of claim 45, wherein said test compound inhibits the interaction of said array of transcription factor targets and said at least one transcription factor polypeptide.

47. The method of claim 45, wherein said test compound enhances the interaction of said array of transcription factor targets and said at least one transcription factor polypeptide.

48. A method for measuring biomolecular interactions, comprising:
a) providing
i) a composition according to claim 4;
ii) a biological sample comprising at least one test compound;
iii) at least one transcription factor binding target; and
iv) a detection apparatus; and
b) contacting said biological sample and said at least one binding target with said arrayed solid surface under conditions such that said detection apparatus detects interactions between said array of transcription factors and said at least one transcription factor binding target.

49. A method for measuring the effect of small molecules on biomolecular interactions, comprising:
a) providing
i) a composition according to claim 1;
ii) at least one small molecule test compound;
iii) a biological sample containing at least one transcription factor;
iv) a detection apparatus; and
b) treating said biological sample with said at least one small molecule test compound to generate a treated biological sample;
c) contacting said treated biological sample with said array of transcription factor targets under conditions such that said apparatus detects interactions between said array of transcription factor targets and said at least one transcription factor contained in said biological sample.
